# EUROPEAN PATENT SPECIFICATION

(11) **EP 3 572 001 B1**
(45) Date of publication and mention of the grant of the patent: **02.06.2021**
(21) Application number: 17892685.3
(22) Date of filing: 23.01.2017
(51) Int. Cl.: A61B 5/00

(54) **CUTANEOUS SENSORY THRESHOLD MEASUREMENT DEVICE**
VORRICHTUNG ZUR MESSUNG DES SENSORISCHEN SCHWELLENWERTS DER HAUT
DISPOSITIF DE MESURE DE SEUIL SENSORIEL CUTANÉ

(43) Date of publication of application: 27.11.2019
(73) Proprietor: Asuka Electric Co. Ltd., Osaka-shi, Osaka 5310041 (JP)
(72) Inventor: YOSHIMURA, Shinichi, Osaka-shi, Osaka 531-0041 (JP)
(74) Representative: Plougmann Vingtoft a/s
(86) International application number: PCT/JP2017/002126
(87) International publication number: WO 2018/134996

(56) References cited:
- JP-A- H09 266 908
- JP-A- 2010 022 585
- US-A- 4 250 891
- US-A- 5 363 859
- US-A1- 2009 099 448
- US-A1- 2012 053 483

## Description

### TECHNICAL FIELD

The present invention relates to a cutaneous sensory measurement device for measuring a cutaneous sensory threshold, in particular, relates to a cutaneous sensory measurement device for measuring a cutaneous sensory threshold of a fingertip of a subject.

### BACKGROUND ART

With regard to this type of measurement device, the applicant has previously proposed a measurement device for measuring a sensory threshold of a sole (Patent Literature 1). The measurement device described in Patent Literature 1 includes a footrest that supports the sole of a sitting or standing subject, a probe that gives a moving stimulus to the sole, a probe drive structure that operates the movement of the probe, an input switch that is operated by the subject who recognizes a sole stimulus, and a drive control unit that controls a drive state of the probe drive structure. According to this measurement device, with the sole placed on a contact window opened at a predetermined position on the footrest, the probe drive structure operates the movement of the probe, and the input switch is operated when the subject feels a moving stimulus on the sole, so that the sensory threshold can be measured.

In addition, according to this measurement device, since the movement of the probe is automatically operated by the probe drive structure to give the moving stimulus to the sole, it is possible to accurately give the subject a moving stimulus as set in a set procedure and to quantitatively measure the sensory threshold with high reproducibility. Such a measurement device is useful for measuring peripheral neuropathy, and it can be expected to be given not only to the evaluation of the degree of nerve recovery after surgery in the field of orthopedic surgery but also to the prediction of the early stage of diabetes with peripheral neuropathy in particular.

US 2009/099448 A1 discloses a system and method are provided for an imaging system that may be used to measure the elasticity of an organ. In the system, an electronic transducer forms an image of a human or animal organ. A mobile part may be moved in translation or rotation, and is arranged to induce the propagation of low frequency vibration in the direction of the organ when actuated. The mobile part delivers the vibrational energy against the human or animal body. The transducer has a fixed part that also emits similar energy. By application of energy to the mobile and fixed parts, and by collection of energy, a control and calculation module may compute parameters of internal organs, such as elasticity.

US 2012/053483 A1 discloses an apparatus for exerting force on a subject tissue includes a linear motor for generating a force according to a predetermined force profile incorporating at least one motion control parameter. The linear motor is directly coupled to a motor output member to drivingly produce linear motion of the motor output member under direction of a motor controller executing the predetermined force profile. A tissue-contacting member is connected to the motor output member for directly proportional linear motion therewith. A load cell provides load cell feedback to the motor controller. The motor controller adjusts the motion of the motor output member responsive to the load cell feedback to substantially conform the motion to the predetermined force profile. The linear motor moves the tissue-contacting member to contact the subject tissue according to the predetermined force profile and responsively initiate a subject reaction to the exerted force.

### CITATIONS LIST

### PATENT LITERATURE

Patent Literature 1: Japanese Unexamined Patent Application Publication No. 2012-223365

### SUMMARY OF INVENTION

### TECHNICAL PROBLEMS

The inventor of the present invention has considered applying the sole cutaneous sensory threshold measurement device described in Patent Literature 1 to measurement of the cutaneous sensory threshold of a fingertip. However, in general, the perceptual sensitivity of the cutaneous sensory of the fingertip is much higher than that of the sole. For this reason, in order to stimulate the cutaneous sensory of the fingertip to measure the cutaneous sensory threshold and accurately evaluate neuropathy of the fingertip, there is a limit in measuring the cutaneous sensory threshold with high reproducibility even if a moving stimulus under the same conditions as the sole is given to the fingertip. More specifically, progress of peripheral neuropathy of the fingertip in the early stage of diabetes is slower than that of the sole, and the perceptual sensitivity of the cutaneous sensory of the fingertip is much higher than that of the sole, and hence it is not easy to recognize a difference in cutaneous sensory threshold between diabetic patients and healthy people only with a moving stimulus to the fingertip and, as a result, it is difficult to appropriately evaluate the presence of peripheral neuropathy and its progress.

An object of the present invention is to provide a cutaneous sensory threshold measurement device that is capable of quantitatively measuring the cutaneous sensory threshold of the human fingertip with high reproducibility, and that is useful in grasping the health condition and identifying the presence of peripheral neuropathy derived from diabetes or the progress of neuropathy.

### SOLUTIONS TO PROBLEMS

The present invention targets a cutaneous sensory measurement device for measuring the cutaneous sensory threshold of a fingertip 10 of a subject. A body case 2 accommodates therein a probe 15 that gives a stimulus by coming into contact with a skin of the fingertip 10, a probe drive unit 16 that drives the probe 15, a drive control unit 20 that controls a drive state of the probe drive unit 16, and a movement amount detection unit 17 that detects a movement amount of the probe 15 moved by the probe drive unit 16. The probe drive unit 16 includes a vibration giving function that gives ultrasonic vibration to the probe 15 and a movement function that moves the probe 15. With the probe 15 in contact with the skin of the fingertip 10, the probe drive unit 16 is driven by a control signal from the drive control unit 20 to give the fingertip 10 a moving stimulus accompanied by an ultrasonic vibration stimulus, and the movement amount detection unit 17 detects the movement amount of the probe 15 at a time when the subject shows a reaction, so that the cutaneous sensory threshold of the fingertip 10 of the subject is measured. With the probe drive unit 16 using an ultrasonic motor 30 as a drive source, the probe 15 is moved by the drive force of the ultrasonic motor 30, and hence ultrasonic vibration derived from the ultrasonic motor 30 is given to the probe 15 and a moving stimulus accompanied by an ultrasonic vibration stimulus is given to the fingertip. The vibration direction of the probe 15 in the present invention is not limited to the direction coinciding with the moving direction (for example, the front-rear direction) of the probe 15 by the movement function, and may be a direction (up-down direction or left-right direction) orthogonal to the moving direction, that is, the vibration sense of the fingertip 10 may be stimulated by the vibration of the probe 15.

It is possible to adopt a form in which the ultrasonic motor 30 configuring the probe drive unit 16 includes a piezoelectric element 32 that is bent and deformed by application of a voltage, a vibration shaft 33 that vibrates in response to the bending and deformation of the piezoelectric element 32, and a moving body 34 that is configured to be movable with respect to the vibration shaft 33 and linearly moves along the vibration shaft 33 in response to the bending and deformation of the piezoelectric element 32, and the probe 15 is fixed to the moving body 34.

It is possible to adopt a form in which the probe drive unit 16 includes two of the vibration shafts 33 arranged in parallel, the piezoelectric element 32 connected to an end portion of each of the vibration shafts 33, the moving body 34 provided on each of the vibration shafts 33, and a table 31 that bridges between both of the moving bodies 34, and the probe 15 is fixed to the table 31.

It is possible to adopt a form in which a contact window 60 for exposing the probe 15 is opened at the central portion of the body case 2, the probe 15 is configured to be movable along the contact window 60, and the contact window 60 is provided with a packing 61 made of an elastic body that seals a gap between an opening edge of the contact window 60 and a peripheral edge of the probe 15.

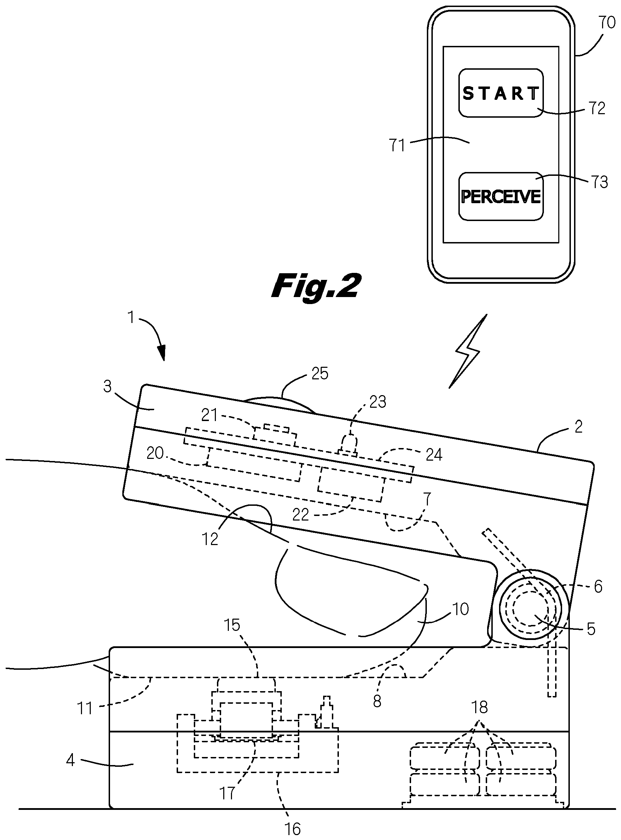

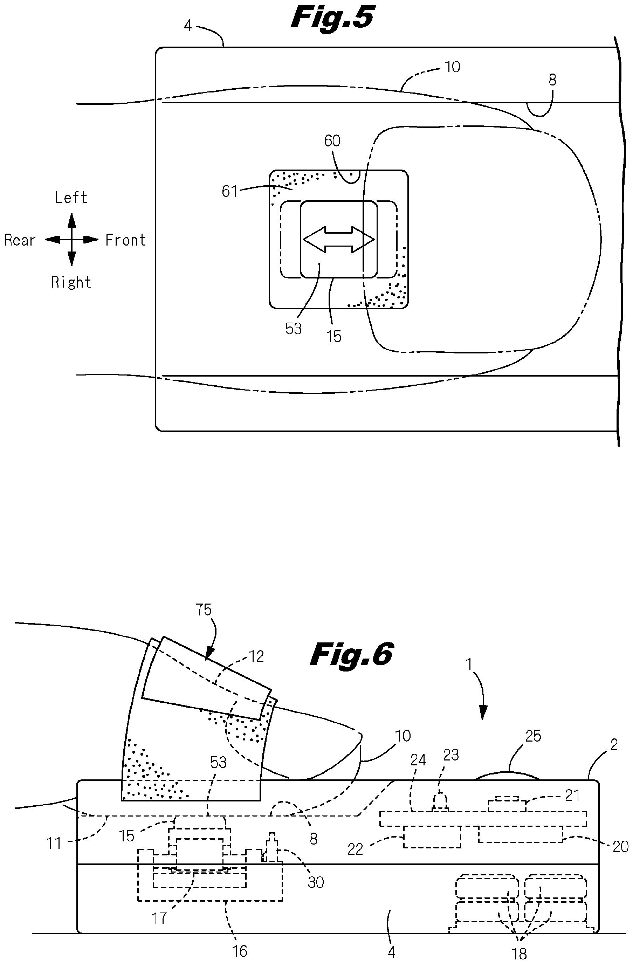

It is possible to adopt a form that includes a mounting structure for mounting and fixing the body case 2 onto the fingertip 10 of the subject, with the probe 15 in contact with the skin through the contact window 60. For the mounting structure, it is possible to adopt a form in which, as shown in FIG. 2 for example, the body case 2 is configured to have a clip shape with an upper case 3, a lower case 4, a connecting shaft 5, a spring 6, and the like and sandwiches the fingertip 10 to be mounted or a form in which, as shown in FIG. 6, the fingertip 10 is tightened with a mounting band 75 to be mounted onto the body case 2.

The body case 2 accommodates therein a communication unit 22 for establishing a wireless communication connection state with a main control device 70 operated by the subject and in which a control program is installed. Based on a control signal transmitted from the main control device 70 via the communication unit 22, the probe 15 is moved and the cutaneous sensory threshold detected by the movement amount detection unit 17 is transmitted toward the main control device 70 via the communication unit 22.

### ADVANTAGEOUS EFFECTS OF INVENTION

In the cutaneous sensory threshold measurement device according to the present invention, the probe 15 not only moves but also ultrasonically vibrates, so that a moving stimulus accompanied by an ultrasonic vibration stimulus is given to the fingertip 10 of the subject. According to this, as compared with the conventional device that gives the subject only a moving stimulus of the probe 15, a combined stimulus can be given to the fingertip 10 and the cutaneous sensory threshold can be measured more appropriately. More specifically, since the vibration sense of the fingertip 10 is stimulated by giving an ultrasonic vibration stimulus and the pressure sense of the fingertip 10 can be stimulated by giving a moving stimulus, further giving an ultrasonic vibration stimulus makes it possible to more appropriately draw a reaction even from a subject who has a poor reaction only with a conventional moving stimulus and, as a result, the sensory threshold of the fingertip 10 can be measured more accurately.

In addition, since the reaction of the subject can be drawn with a smaller movement amount of the probe 15 as compared with the conventional device that gives only a moving stimulus, the measurement device can be miniaturized by preventing the movement mechanism of the probe 15 from becoming unnecessarily large in size. Therefore, according to the present invention, it is possible to miniaturize the device and to obtain a small and lightweight measurement device appropriate for measuring the sensory threshold of the fingertip 10.

In a conventional device that only gives a moving stimulus, it may be difficult for, in particular, a subject with peripheral neuropathy and decreased perceptual sensitivity to appropriately perceive even the contact state and the contact position of the probe 15 in an initial state where the probe 15 is in contact with the human skin. Even if the probe 15 is moved in units of several µm from the initial state and a moving stimulus is given to such a subject, the perceptual sensitivity in the initial state is vague in the first place, and hence it is impossible for the subject to appropriately react the movement of the probe 15 and it is likely to become difficult to measure the cutaneous sensory threshold. On the other hand, as in the measurement device according to the present invention, a configuration in which a moving stimulus accompanied by an ultrasonic vibration stimulus is given to the human skin allows the subject to appropriately perceive the contact state and the contact position of the probe 15 in the initial state by giving an ultrasonic vibration stimulus to stimulate the vibration sense of the fingertip 10 in the initial state where the probe 15 is brought into contact with the human skin of the subject. Also, by moving the probe 15 from the initial state and giving a moving stimulus accompanied by an ultrasonic vibration stimulus to the human skin, the subject can appropriately respond to the movement of the probe 15. Also in the above points, use of the measurement device according to the present invention enables a cutaneous sensory threshold to be quantitatively measured with higher reproducibility.

With the probe drive unit 16 using the ultrasonic motor 30 as a drive source, the probe 15 is moved by the drive force of the ultrasonic motor 30, and hence ultrasonic vibration derived from the ultrasonic motor 30 is given to the probe 15 and a moving stimulus accompanied by an ultrasonic vibration stimulus is given to the fingertip 10. According to this, the structure of the probe drive unit 16 can be simplified as compared with the configuration in which the vibration giving function for ultrasonically vibrating the probe 15 and the movement function for moving the probe 15 are realized as separate mechanisms, and therefore it is possible to obtain a cutaneous sensory threshold measurement device preferable for miniaturizing the entire device.

It is possible to adopt a form in which the ultrasonic motor 30 configuring the probe drive unit 16 includes a piezoelectric element 32 that is bent and deformed by application of a voltage, a vibration shaft 33 that vibrates in response to the bending and deformation of the piezoelectric element 32, and a moving body 34 that is configured to be movable with respect to the vibration shaft 33 and linearly moves along the vibration shaft 33 in response to the bending and deformation of the piezoelectric element 32, and the probe 15 is fixed to the moving body 34. The ultrasonic motor 30 as described above is excellent in exerting a large drive force for its size and being capable of realizing a stable operation with a high operation speed. Therefore, it is preferable as a drive source for the small and lightweight cutaneous sensory threshold measurement device for the fingertip 10.

It is possible to adopt a form in which the probe drive unit 16 includes two of the vibration shafts 33 arranged in parallel, the piezoelectric element 32 connected to the end portion of each of the vibration shafts 33, the table 31 that bridges between the two vibration shafts 33, and the probe 15 fixed to the table 31. In other words, it is possible to adopt a form in which the two ultrasonic motors 30 are arranged in parallel. According to this, since a load applied to the probe 15 can be received by the two vibration shafts 33, it is possible to obtain a cutaneous sensory threshold measurement device excellent in durability with less breakage of the vibration shaft 33. Since the probe 15 is moved by the two ultrasonic motors 30, it is possible to reliably move the probe 15 even when the probe 15 is strongly pressed, thereby allowing a measurement device excellent in reliability to be obtained.

It is possible to adopt a form in which the contact window 60 for exposing the probe 15 is opened at the central portion of the body case 2, the probe 15 is configured to be movable along the contact window 60, and the packing 61 made of an elastic body that seals a gap between an opening edge of the contact window 60 and a peripheral edge of the probe 15 is arranged. According to this, it is possible to seal the gap between the probe 15 and the contact window 60 with the packing 61 and to prevent dust, moisture, and the like from entering the body case 2 through the contact window 60. Therefore, it is possible to prevent malfunction of the probe drive unit 16 and the like from occurring and to contribute to improvement of reliability of the device.

If provided with the mounting structure for mounting and fixing the body case 2 onto the fingertip 10 of the subject, with the probe 15 in contact with the skin through the contact window 60, it is possible to prevent the fingertip 10 of the subject from unintentionally moving with respect to the body case 2, and hence it is possible to more accurately measure the cutaneous sensory threshold of the fingertip 10 of the subject.

It is possible to adopt a form in which the body case 2 accommodates therein the communication unit 22 for wireless communication, and the measurement device is connected via the communication unit 22 with the main control device 70 operated by the subject. As described above, with the measurement device being provided with the communication unit 22 for wireless communication with the main control device 70, it is possible to adopt a form in which, for example, the main control device 70 is provided with a switch and, if the switch of the main control device 70 is operated when the subject feels a moving stimulus on the fingertip 10, the movement amount detection unit 17 measures the cutaneous sensory threshold and transmits the cutaneous sensory threshold toward the main control device 70 via the communication unit 22. According to this, the configuration of the measurement device can be simplified as compared with a configuration in which a switch or the like operated when the subject feels a moving stimulus on the fingertip 10 is provided on the measurement device side. Further, the configuration of the measurement device can be simplified because it is possible to adopt a configuration in which the control program installed in advance in the main control device 70 causes the main control device 70 to automatically determine the presence of neuropathy and the degree of the neuropathy in the fingertip 10 or a configuration in which a determination result or the like is displayed on display means provided in the main control device 70. If the measurement device and the main control device 70 are configured to be connected via wireless communication therebetween, it is possible to dramatically more easily establish a connection state between the both as compared with a case of connecting the measurement device with the main control device 70 via a wire. Since the measurement device and the main control device 70 are connected via wireless communication, the operation of the main control device 70 does not have the disadvantage of being dragged by the wire and causing the measurement device to slip, and has the advantage of more accurately measuring the cutaneous sensory threshold.

### BRIEF DESCRIPTION OF DRAWINGS

FIG. 1 is a longitudinal sectional side view showing a main portion of a cutaneous sensory threshold measurement device according to the present invention.
FIG. 2 is a view showing an entire configuration of the cutaneous sensory threshold measurement device.
FIG. 3 is a sectional view taken along A-A line of FIG. 1.
FIG. 4 is a plan view of a main portion of the cutaneous sensory threshold measurement device.
FIG. 5 is a view for describing an operation of a probe.
FIG. 6 is a view showing another embodiment of a cutaneous sensory threshold measurement device according to the present invention.

### DESCRIPTION OF EMBODIMENTS

### (Embodiment)

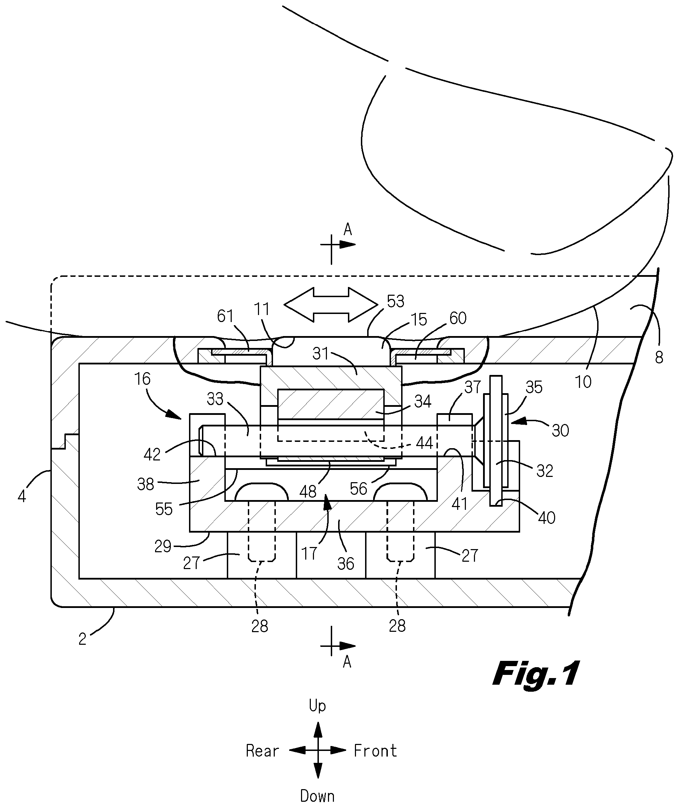

FIGS. 1 to 5 show an embodiment of a cutaneous sensory threshold measurement device according to the present invention. Front and rear, right and left, and upper and lower in the present embodiment follow the crossing arrows shown in FIGS. 1, 3, and 5 and the writing of front and rear, right and left, and upper and lower in the vicinity of the crossing arrows.

As shown in FIG. 2, a body case 2 serving as a base of a measurement device 1 has a clip shape and includes upper case 3 and lower case 4 each formed in a quadrangular container shape, connecting shaft 5 connecting between the upper and lower cases 3 and 4, and torsion coil spring 6 mounted on the connecting shaft 5. The measurement device 1 is configured to be swingable between a closed state in which the facing distance between the upper case 3 and the lower case 4 becomes small with the connecting shaft 5 as a center by the biasing force of the spring 6 and an open state in which the facing distance between the upper case 3 and the lower case 4 becomes large against the biasing force of the spring 6. In measurement, the upper case 3 is opened to set in the open state, a fingertip 10 (for example, thumb) to be measured is inserted between the upper case 3 and the lower case 4, the upper case 3 is closed by the biasing force of the spring 6, and the fingertip 10 is sandwiched (clipped) from the up-down direction between the upper case 3 and the lower case 4 as shown in FIG. 2, and thus the measurement device 1 can be mounted onto the fingertip 10. In addition, the measurement device 1 can be removed from the fingertip 10 by setting the upper case 3 in the open state against the biasing force of the spring 6 from the mounting state described above.

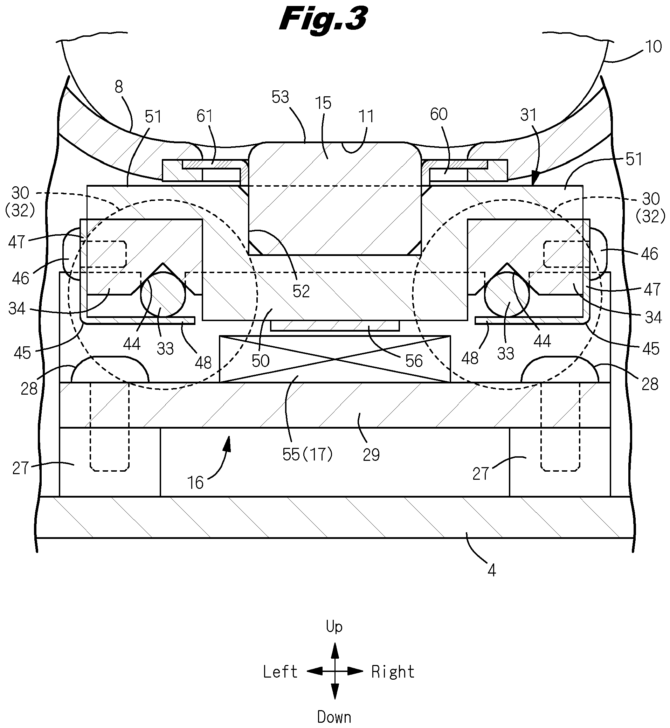

As shown in FIGS. 2 and 3, concave surfaces 7 and 8 serving as guides for the fingertip 10 are formed on the lower surface of the upper case 3 and the upper surface of the lower case 4, respectively. With a front 11 of the fingertip 10 being pressed along the concave surface 8 of the lower case 4, by putting the concave surface 7 of the upper case 3 on a back 12 of the fingertip 10, the measurement device 1 can be mounted onto an optimum position of the fingertip 10. In the present embodiment, the mounting structure is configured by the body case 2 having a clip shape with the upper case 3, the lower case 4, the connecting shaft 5, the spring 6, and the like.

The lower case 4 accommodates therein a probe 15 that comes into contact with the skin of the front 11 of the fingertip 10 to give a stimulus, a probe drive unit 16 that drives the probe 15, a movement amount detection unit 17 that detects a movement amount of the probe 15, and a battery 18 that supplies electric power to various members such as the probe drive unit 16. The upper case 3 accommodates therein a control chip (drive control unit) 20 of the measurement device 1, a power switch 21, a wireless communication chip (communication unit) 22, and a circuit board 24 on which a light-emitting element 23 for displaying a measurement state and the like are mounted. In FIG. 1, a reference numeral 25 denotes an operation button for turning the power switch 21 on and off.

As shown in FIGS. 1, 3, and 4, the probe drive unit 16 is configured with a drive base 29 fastened and fixed by a screw 28 to a screw boss 27 erected at four positions on the bottom surface of the lower case 4, a pair of left and right ultrasonic motors 30, 30 supported by the drive base 29, and a table 31 slidably guided in the front-rear direction by the ultrasonic motors 30, 30. Each of the ultrasonic motors 30 is configured with a piezoelectric element 32 that is bent and deformed by application of a voltage, a vibration shaft 33 that vibrates in response to the deformation of the piezoelectric element 32, and a moving body 34 that is configured to be movable with respect to the vibration shaft 33 and linearly moves along the vibration shaft 33 in response to the bending and deformation of the piezoelectric element 32. The piezoelectric element 32 includes an electrostrictive board 35 that is bent and deformed by application of a voltage.

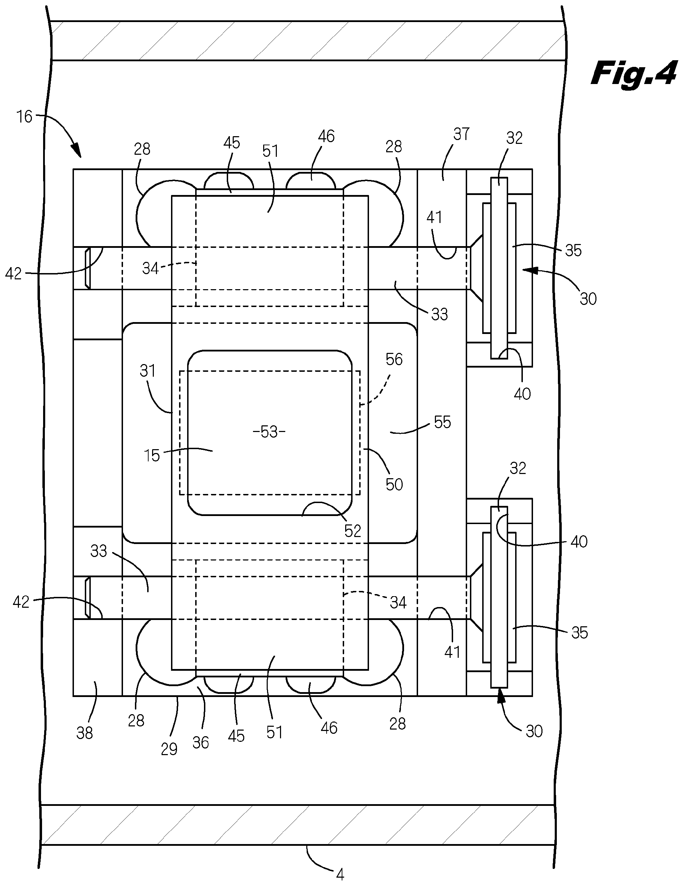

As shown in FIGS. 1 and 4, the drive base 29 is a resin molded item that includes a quadrangular plate-shaped base main body 36 having a through hole for the screw 28, and a pair of front and rear support walls 37 and 38 provided at the front and rear ends, respectively, of the base main body 36. A pair of left and right support structures that supports the ultrasonic motors 30, 30 is formed on the support walls 37 and 38, respectively. Each of the support structures is configured with a mounting groove 40 formed at the front end of the base main body 36 of the drive base 29 to fixedly hold the lower end portion of a casing of the piezoelectric element 32 of the ultrasonic motor 30 in an outer fitting manner, a front bearing 41 formed on the front support wall 37 to receive a base end portion of the vibration shaft 33, and a rear bearing 42 formed on the rear support wall 38 to receive a tip portion of the vibration shaft 33. The bearings 41 and 42 are grooves formed in a concave manner on the upper end surfaces of the front and rear support walls 37 and 38, respectively.

The moving body 34 is a quadrangular pillar shaped metal molded item mounted onto a middle portion of the vibration shaft 33 in the front-rear direction.
The lower surface of the moving body 34 has a receiving concave portion 44 with an opening facing downward that has an angled cross section that is received at the upper portion of the vibration shaft 33 (see FIG. 3). A sandwiching body 45 is fixed in the moving body 34. The sandwiching body 45 is arranged opposite to the receiving concave portion 44 to sandwich the vibration shaft 33 from the up-down direction in cooperation with the receiving concave portion 44 and generate an appropriate frictional force between the moving body 34 and the vibration shaft 33. The sandwiching body 45 is a metal molded item having an L-shaped cross section that includes a support piece 47 fixed to the side surface of the moving body 34 by a screw 46, and an elastic piece 48 extending horizontally from the lower end of the support piece 47. Contact of the upper surface of the free end of the elastic piece 48 with the lower portion of the vibration shaft 33 prevents the moving body 34 from being largely lifted from the vibration shaft 33. From the above, together with the receiving concave portion 44 having an angled cross section and the moving body 34 brought into a linear contact with the vibration shaft 33, it is possible to provide an appropriate frictional force generated between the moving body 34 and the vibration shaft 33. Hence, it is possible to appropriately control the movement of the moving body 34 along the vibration shaft 33 according to the law of inertia when the vibration shaft 33 vibrates axially.

More specifically, in the ultrasonic motor 30 configured as described above, by giving a pulse wave to the piezoelectric element 32, it is possible to deform the state of the electrostrictive board 35 configuring the piezoelectric element 32 between a neutral state and a bending and deformation state and to vibrate the vibration shaft 33. At this time, for example, by giving a sawtooth pulse wave to the piezoelectric element 32, it is possible to increase the speed of "return" from the bending and deformation state to the neutral state of the electrostrictive board 35 by a restoring force of the electrostrictive board 35 itself than the speed of "outward" from the neutral state to the bending and deformation state of the electrostrictive board 35. Hence, the moving body 34 moving on the vibration shaft 33 according to the law of inertia is left by a minute amount and, as a result, the moving body 34 can be moved linearly along the vibration shaft 33. The ultrasonic motor 30 in the present embodiment vibrates at 30 to 80 kHz.

The table 31 is a block-shaped resin molded item that includes a probe fixing portion 50 at the center and moving body fixing portions 51, 51 that are formed so as to protrude on the both left and right sides of the probe fixing portion 50. A mounting hole 52 having a square shape in a plan view is formed at the center of the probe fixing portion 50, and the probe 15 can be moved along with the movement of the table 31 by fitting the probe 15 into the mounting hole 52. The moving body 34 is adhesively fixed to the lower surface of each of the moving body fixing portions 51. The probe 15 is a quadrangular pillar shaped resin molded item to give a stimulus to the front 11 of the fingertip 10 with a flat contact portion 53 provided on the upper end thereof. The contact portion 53 is formed in a square of 5 mm × 5 mm in a plan view.

The movement amount detection unit 17 that detects the movement amount of the probe 15 is a noncontact linear scale including a position detection sensor 55 provided on the upper surface of the drive base 29 and a magnetic scale 56 provided on the lower surface of the table 31. The movement amount of the probe 15 fixed on the table 31 is detected by capturing the position of the magnetic scale 56 using the position detection sensor 55. The detection result by the position detection sensor 55 is sent to the control chip 20 disposed on the circuit board 24 via a line not illustrated.

As shown in FIGS. 1, 3, and 5, a contact window 60 for exposing the probe 15 is opened at the center of the upper surface of the lower case 4. The dimension of the opening of the contact window 60 is 10 mm × 10 mm. The contact portion 53 of the probe 15 is exposed on the upper surface through the contact window 60 to come into contact with the front 11 of the fingertip 10. In order to prevent dust, moisture, and the like from entering the inside of the body case 2 through the contact window 60, a packing 61 is disposed between the opening peripheral edge of the contact window 60 and the peripheral edge of the probe 15. As shown in FIG. 5, the packing 61 is a rubber molded item having a quadrangular shape in a plan view that includes an opening of about 5 mm × 5 mm matching the outer shape of the probe 15, and, as shown in FIG. 3, the packing 61 is fixed to the probe fixing portion 50 in a state where the four peripheral edge of the packing 61 are joined to the opening peripheral edge of the contact window 60 and the opening peripheral edge of the packing 61 is in contact with the peripheral edge of the probe 15. Thus, regardless of the movement position of the probe 15, the gap between the contact window 60 and the probe 15 can constantly be sealed with the packing 61, so that it is possible to surely prevent dust and the like from entering the body case 2. Therefore, it is possible to prevent malfunction of the probe drive unit 16 and the like from occurring and to contribute to improvement of reliability of the device.

The drive control unit, which is the control chip 20 mounted onto the circuit board 24, controls the drive state of the probe drive unit 16, and moves the probe 15 in the front-rear direction in a predetermined procedure. The moving speed of the probe 15 by the probe drive unit 16 can be set in increments of 1 mm/s, and the moving distance can be set in increments of 2 µm.

A main control device 70 operated by a subject is an information portable terminal in which a control program is installed in advance, and establishes a wireless communication connection state with a wireless communication chip 22 of the measurement device 1 using a short-distance wireless communication standard such as IEEE 802.15.1. When the control program is launched, a start button 72, a sensing button 73, and the like are displayed on a touch-panel display screen 71 of the main control device 70. The sensing button 73 is turned on when the subject feels a moving stimulus in the process of measuring the sensory threshold with the measurement device 1, and an ON signal when the sensing button 73 is turned on is stored in a recording unit of the main control device 70 together with the movement situation of the probe 15. When the control program is installed in the main control device 70, information such as the age and sex of the subject is registered, and the subject information is also recorded in the recording unit.

The method for measuring the sensory threshold of the fingertip using the measurement device with the above configuration will be described. First, the measurement device 1 is mounted onto the fingertip 10 by clipping the fingertip 10 from the up-down direction with the upper case 3 and the lower case 4 as shown in FIG. 2. At this time, the measurement device 1 is mounted at an appropriate position where the probe 15 comes in contact with the front 11 of the fingertip 10.

Next, the control program of the main control device 70 is launched. At this time, the ON signal is transmitted from the main control device 70 to the measurement device 1 via wireless communication, whereby the measurement device 1 is operated and the wireless connection state between the main control device 70 and the measurement device 1 is established. A wireless connection state may be established with the main control device 70 via wireless communication when the operation button 25 of the measurement device 1 is pressed to turn on the power switch 21 and the measurement device 1 is operated. The point is that the wireless connection state between the main control device 70 and the measurement device 1 may be established prior to the measurement with the measurement device 1.

Next, when the start button 72 of the main control device 70 is turned on, the measurement device 1 operates the probe drive unit 16 to cause the probe 15 to reciprocate in the front-rear direction with micro-vibration to give a moving stimulus accompanied by an ultrasonic vibration stimulus to the front 11 of the fingertip 10. Here, the probe 15 is moved at a predetermined speed (for example, a speed of 5 mm/s) and a predetermined width (for example, a width of 40 µm). In the meantime, if the subject turns on the sensing button 73 of the main control device 70, a moving stimulus accompanied by a small ultrasonic vibration stimulus that is even less likely to be perceived is given to the subject so as to confirm whether the subject has been able to perceive the movement of the probe 15. In addition, when there is no reaction from the subject even if a moving stimulus is given, the probe 15 is moved with a large width that is more likely to be perceived so as to confirm whether the subject has been able to perceive a moving stimulus accompanied by an ultrasonic vibration. By measuring the moving stimulus accompanied by the ultrasonic vibration with variable magnitudes in this manner, the cutaneous sensory threshold of the fingertip 10 of the subject to the moving stimulus accompanied by the ultrasonic vibration in the front-rear direction can be measured quantitatively with high reproducibility.

When the cutaneous sensory threshold is measured as described above, the main control device 70 compares the measurement result with a database constructed based on age and sex, evaluates the degree of progress of peripheral neuropathy, and displays the evaluation result on the display screen 71 together with the measurement result described above. This allows even a subject without specialized knowledge and skills to reflect an obtained result into prevention of diabetes and the like. Since the perceptual state of the cutaneous sensory of the fingertip 10 can be objectively grasped, the obtained measurement result can also be used as a barometer for measuring the mental state and the health state of the subject. That is, this measurement device 1 can be preferably used not only for the purpose of preventing a disease such as diabetes but also for the purpose of measuring the mental state or health state of the subject. The measurement result obtained as described above is accumulated in the recording unit of the main control device 70 together with the time information, and the measurement result and the evaluation result can be displayed on the display screen 71 graphically.

FIG. 6 shows another embodiment of the measurement device, in which the mounting structure is different. The upper case 3 is omitted in the body case 2, and the mounting structure is configured with the lower case 4 and a mounting band 75 provided on the lower case 4. The mounting band 75 is formed of a pair of left and right band cloths including male and female hook and loop fasteners, and ends of the band cloths are fixed to the left and right side surfaces of the lower case 4 respectively. The measurement device 1 can be mounted onto the fingertip 10 by placing the front 11 of the fingertip 10 on the concave surface 8 and bringing the male and female hook and loop fasteners of the mounting band 75 into close contact and tightening on the back 12 side. The circuit board 24 and the like disposed on the upper case 3 are disposed in the lower case 4. Since the other parts are the same as those in the previous embodiment, the same reference numerals are given to the same members and the description thereof will be omitted.

As described above, in the cutaneous sensory threshold measurement device according to each of the above-described embodiments, the probe 15 not only moves but also ultrasonically vibrates, so that a moving stimulus accompanied by an ultrasonic vibration stimulus is given to the fingertip 10 of the subject, and therefore, compared with the conventional device that gives the subject only a moving stimulus of the probe 15, a combined stimulus can be given to the fingertip 10 and the cutaneous sensory threshold can be measured more appropriately. More specifically, since the vibration sense of the fingertip 10 is stimulated by giving an ultrasonic vibration stimulus and the pressure sense of the fingertip 10 can be stimulated by giving a moving stimulus, further giving an ultrasonic vibration stimulus makes it possible to more appropriately draw a reaction even from a subject who has a poor reaction only with a conventional moving stimulus and, as a result, the sensory threshold of the fingertip 10 can be measured more accurately.

In addition, since the reaction of the subject can be drawn with a smaller movement amount of the probe 15 as compared with the conventional device that gives only a moving stimulus, the measurement device 1 can be miniaturized by preventing the movement mechanism of the probe 15 from becoming unnecessarily large in size. As mentioned above, it is possible to miniaturize the device and to obtain a small and lightweight measurement device 1 appropriate for measuring the sensory threshold of the fingertip 10.

As in the measurement device according to each of the above-described embodiments, since the moving stimulus accompanied by the ultrasonic vibration stimulus is given to the human skin, it becomes possible to allow the subject to appropriately perceive the contact state and the contact position of the probe 15 in the initial state by giving an ultrasonic vibration stimulus in the initial state where the probe 15 is brought into contact with the human skin of the subject. Also, by moving the probe 15 from the initial state and giving a moving stimulus accompanied by an ultrasonic vibration stimulus to the human skin, the subject can appropriately respond to the movement of the probe 15, and it becomes possible to quantitatively measure the cutaneous sensory threshold with higher reproducibility.

Since the probe drive unit 16 moves the probe 15 by the drive force of the ultrasonic motor 30, ultrasonic vibration derived from the ultrasonic motor 30 is given to the probe 15, and a moving stimulus accompanied by an ultrasonic vibration stimulus is given to the fingertip, the structure of the probe drive unit 16 can be simplified as compared with the configuration in which the vibration giving function for ultrasonically vibrating the probe 15 and the movement function for moving the probe 15 are realized as separate mechanisms. Accordingly, it is possible to obtain a cutaneous sensory threshold measurement device 1 preferable for miniaturizing the entire device.

If the probe 15 is fixed to the moving body 34 of the ultrasonic motor 30 including the piezoelectric element 32, the vibration shaft 33, and the moving body 34, the ultrasonic motor 30 as described above is excellent in exerting a large drive force for its size and being capable of realizing a stable operation with a high operation speed. Therefore, it is preferable as a drive source for the small and lightweight cutaneous sensory threshold measurement device 1 for the fingertip 10.

According to the probe drive unit 16 in a form where the two ultrasonic motors 30 are arranged in parallel, since a load applied to the probe 15 can be received by the two vibration shafts 33, it is possible to obtain the cutaneous sensory threshold measurement device 1 excellent in durability with less breakage of the vibration shaft 33. Since the probe 15 is moved by the two ultrasonic motors 30, it is possible to reliably move the probe 15 even when the probe 15 is strongly pressed, thereby allowing a measurement device 1 excellent in reliability to be obtained.

Since provided with the mounting structure for mounting and fixing the body case 2 onto the fingertip 10 of the subject, with the probe 15 in contact with the skin through the contact window 60, it is possible to prevent the fingertip 10 of the subject from unintentionally moving with respect to the body case 2, and it is possible to more accurately measure the cutaneous sensory threshold of the fingertip 10 of the subject.

The measurement device 1 is provided with the wireless communication chip 22 for wireless communication with the main control device 70, the main control device 70 is provided with the sensing button 73, the movement amount detection unit 17 measures the cutaneous sensory threshold if the sensing button 73 of the main control device 70 is operated when the subject feels a moving stimulus on the fingertip 10, and the cutaneous sensory threshold is transmitted toward the main control device 70 via the wireless communication chip 22. According to this, the configuration of the measurement device 1 can be simplified as compared with a configuration in which the sensing button 73 operated when the subject feels a moving stimulus on the fingertip 10 is provided on the measurement device 1 side. Further, the configuration of the measurement device 1 can be simplified because it is possible to adopt a configuration in which the control program installed in advance in the main control device 70 causes the main control device 70 to automatically determine the presence of neuropathy and the degree of the neuropathy in the fingertip 10 or a configuration in which a determination result or the like is displayed on the display screen 71 provided in the main control device 70. If the measurement device 1 and the main control device 70 are configured to be connected via wireless communication therebetween, it is possible to dramatically more easily establish a connection state between the both as compared with a case of connecting the measurement device 1 with the main control device 70 via a wire. Since the measurement device 1 and the main control device 70 are connected via wireless communication, the operation of the main control device 70 does not have the disadvantage of being dragged by the wire and causing the measurement device 1 to slip, and has the advantage of more accurately measuring the cutaneous sensory threshold.

In each of the above-described embodiments, it is configured that a moving stimulus accompanied by an ultrasonic vibration stimulus is given to the fingertip 10 using the ultrasonic motor 30. However, in an alternative construction not part of the present invention, the probe 15 may be moved by a stepping motor and a mechanism that converts a rotational movement of a ball screw and the like into a linear movement and an ultrasonic vibration may be given to the probe 15 by an ultrasonic oscillator fixed to the table 31. The mounting structure may be a stretchable band body fixed so as to cross the upper surface of the lower case 4 instead of the mounting band 75.

### REFERENCE SGNS LIST

- 2: Body case
- 10: Fingertip
- 15: Probe
- 16: Probe drive unit
- 17: Movement amount detection unit
- 20: Drive control unit (Control chip)
- 22: Communication unit (Wireless communication chip)
- 30: Ultrasonic motor
- 31: Table
- 32: Piezoelectric element
- 33: Vibration shaft
- 34: Moving body
- 60: Contact window
- 61: Packing
- 70: Main control device

## Claims

1. A cutaneous sensory threshold measurement device for measuring a cutaneous sensory threshold of a fingertip (10) of a subject, said device comprising:
a body case (2) accommodating therein a probe (15) configured to give a stimulus by coming into contact with a skin of the fingertip (10), a probe drive unit (16) configured to drive the probe (15), a drive control unit (20) configured to control a drive state of the probe drive unit (16), and a movement amount detection unit (17) configured to detect a movement amount of the probe (15) moved by the probe drive unit (16);
the probe drive unit (16) includes a vibration giving function configured to give ultrasonic vibration to the probe (15) and a movement function configured to move the probe (15); and
with the probe (15) in contact with the skin of the fingertip (10), the probe drive unit (16) is driven by a control signal from the drive control unit (20) to give the fingertip (10) a moving stimulus accompanied by an ultrasonic vibration stimulus, and the movement amount detection unit (17) is configured to detect a movement amount of the probe (15) at a time when the subject shows a reaction, so that a cutaneous sensory threshold of the fingertip (10) of the subject is measured,
wherein
the probe drive unit (16) uses an ultrasonic motor (30) as a drive source; and
the probe (15) is moved by a drive force of the ultrasonic motor (30), so that ultrasonic vibration derived from the ultrasonic motor (30) is given to the probe (15), and a moving stimulus accompanied by an ultrasonic vibration stimulus is given to the fingertip (10).

2. The cutaneous sensory threshold measurement device according to claim 1, wherein:
the ultrasonic motor (30) configuring the probe drive unit (16) includes a piezoelectric element (32) that is bent and deformed by application of a voltage, a vibration shaft (33) that vibrates in response to bending and deformation of the piezoelectric element (32), and a moving body (34) that is configured to be movable with respect to the vibration shaft (33) and linearly moves along the vibration shaft (33) in response to bending and deformation of the piezoelectric element (32); and
the probe (15) is fixed to the moving body (34).

3. The cutaneous sensory threshold measurement device according to claim 2, wherein:
the probe drive unit (16) includes two of the vibration shafts (33) arranged in parallel, the piezoelectric element (32) connected to an end portion of each of the vibration shafts (33), the moving body (34) provided on each of the vibration shafts (33), and a table 31 that bridges between both of the moving bodies (34); and
the probe (15) is fixed to the table (31).

4. The cutaneous sensory threshold measurement device according to any one of claims 1 to 3, wherein:
a contact window (60) for exposing the probe (15) is opened at a central portion of the body case (2), and the probe (15) is configured to be movable along the contact window (60); and
the contact window (60) is provided with a packing (61) made of an elastic body that seals a gap between an opening edge of the contact window (60) and a peripheral edge of the probe (15).

5. The cutaneous sensory threshold measurement device according to claim 4, comprising a mounting structure for mounting and fixing the body case (2) onto the fingertip (10) of the subject, with the probe (15) in contact with a skin through the contact window (60).

6. The cutaneous sensory threshold measurement device according to any one of claims 1 to 5, wherein:
the body case (2) accommodates therein a communication unit (22) for establishing a wireless communication connection state with a main control device (70) operated by the subject and in which a control program is installed;
based on a control signal transmitted from the main control device (70) via the communication unit (22), the probe (15) is moved; and
a cutaneous sensory threshold detected by the movement amount detection unit (17) is transmitted toward the main control device (70) via the communication unit (22).

## Patentansprüche

1. Vorrichtung zur Messung des sensorischen Schwellenwerts der Haut zum Messen eines sensorischen Schwellenwerts der Haut einer Fingerspitze (10) einer Person, wobei die Vorrichtung umfasst
ein Körpergehäuse (2), in dem eine Sonde (15) untergebracht ist, die so konfiguriert ist, dass sie einen Reiz abgibt, indem sie mit einer Haut der Fingerspitze (10) in Kontakt kommt, eine Sondenantriebseinheit (16), die so konfiguriert ist, dass sie die Sonde (15) antreibt, eine Antriebssteuereinheit (20), die so konfiguriert ist, dass sie einen Antriebszustand der Sondenantriebseinheit (16) steuert, und eine Bewegungsausmaßerfassungseinheit (17), die so konfiguriert ist, dass sie ein Bewegungsausmaß der durch die Sondenantriebseinheit (16) bewegten Sonde (15) erfasst;
die Sondenantriebseinheit (16) schließt eine Vibrationsabgabefunktion, die konfiguriert ist, um Ultraschallvibrationen an die Sonde (15) abzugeben, und eine Bewegungsfunktion ein, die konfiguriert ist, um die Sonde (15) zu bewegen; und
die Sondenantriebseinheit (16), wenn die Sonde (15) mit der Haut der Fingerspitze (10) in Kontakt ist, wird durch ein Steuersignal von der Antriebssteuereinheit (20) angesteuert, um der Fingerspitze (10) einen Bewegungsreiz zu geben, der von einem Ultraschallvibrationsreiz begleitet wird, und die Bewegungsausmaßerfassungseinheit (17) konfiguriert ist, um ein Bewegungsausmaß der Sonde (15) zu einem Zeitpunkt zu erfassen, wenn die Person eine Reaktion zeigt, sodass ein sensorischer Schwellenwert der Haut der Fingerspitze (10) der Person gemessen wird,
wobei
die Sondenantriebseinheit (16) einen Ultraschallmotor (30) als Antriebsquelle verwendet; und
die Sonde (15) durch eine Antriebskraft des Ultraschallmotors (30) bewegt wird, sodass eine von dem Ultraschallmotor (30) abgeleitete Ultraschallvibration auf die Sonde (15) gegeben wird und ein Bewegungsreiz, der von einem Ultraschallvibrationsreiz begleitet wird, auf die Fingerspitze (10) gegeben wird.

2. Vorrichtung zur Messung des sensorischen Schwellenwerts der Haut nach Anspruch 1, wobei:
der Ultraschallmotor (30), der die Sondenantriebseinheit (16) konfiguriert, ein piezoelektrisches Element (32), das durch Anlegen einer Spannung gebogen und verformt wird, eine Vibrationswelle (33), die in Reaktion auf die Biegung und Verformung des piezoelektrischen Elements (32) vibriert, und einen Bewegungskörper (34) einschließt, der so konfiguriert ist, dass er in Bezug auf die Vibrationswelle (33) beweglich ist und sich in Reaktion auf die Biegung und Verformung des piezoelektrischen Elements (32) linear entlang der Vibrationswelle (33) bewegt; und
die Sonde (15) an dem beweglichen Körper (34) befestigt ist.

3. Vorrichtung zur Messung des sensorischen Schwellenwerts der Haut nach Anspruch 2, wobei:
die Sondenantriebseinheit (16) zwei der Vibrationswellen (33), die parallel angeordnet sind, das piezoelektrische Element (32), das mit einem Endabschnitt jeder der Vibrationswellen (33) verbunden ist, den bewegliche Körper (34), der auf jeder der Vibrationswellen (33) bereitgestellt ist, und ein Tisch (31) einschließt, der zwischen den beiden beweglichen Körpern (34) eine Brücke bildet; und
die Sonde (15) an dem Tisch (31) befestigt ist.

4. Vorrichtung zur Messung des sensorischen Schwellenwerts der Haut nach einem der Ansprüche 1 bis 3, wobei:
ein Kontaktfenster (60) zum Freilegen der Sonde (15) an einem zentralen Abschnitt des Körpergehäuses (2) geöffnet ist, und die Sonde (15) so konfiguriert ist, dass sie entlang des Kontaktfensters (60) bewegbar ist; und
das Kontaktfenster (60) mit einer Dichtung (61) bereitgestellt ist, die aus einem elastischen Körper besteht, der einen Spalt zwischen einem Öffnungsrand des Kontaktfensters (60) und einem Umfangsrand der Sonde (15) abdichtet.

5. Vorrichtung zur Messung des sensorischen Schwellenwerts der Haut nach Anspruch 4, umfassend eine Montagestruktur zum Montieren und Befestigen des Körpergehäuses (2) an der Fingerspitze (10) der Person, wobei die Sonde (15) durch das Kontaktfenster (60) in Kontakt mit der Haut steht.

6. Vorrichtung zur Messung des sensorischen Schwellenwerts der Haut nach einem der Ansprüche 1 bis 5, wobei:
das Körpergehäuse (2) darin eine Kommunikationseinheit (22) zum Herstellen eines drahtlosen Kommunikationsverbindungszustandes mit einem Hauptsteuervorrichtung (70) aufnimmt, das von der Person bedient wird und in dem ein Steuerprogramm installiert ist;
die Sonde (15) basierend auf einem Steuersignal, das von der Hauptsteuervorrichtung (70) über die Kommunikationseinheit (22) übertragen wird, bewegt wird; und
ein sensorischer Schwellenwert der Haut, der von der Bewegungsausmaßerfassungseinheit (17) erfasst wird, über die Kommunikationseinheit (22) an die Hauptsteuervorrichtung (70) übertragen wird.

## Revendications

1. Dispositif de mesure de seuil sensoriel cutané, destiné à mesurer un seuil sensoriel cutané d'un bout de doigt (10) d'un sujet, ledit dispositif comprenant
un boîtier de corps (2) logeant en son sein une sonde (15) configurée pour fournir un stimulus en entrant en contact avec une peau de bout de doigt (10), une unité d'entraînement (16) de sonde configurée pour entraîner la sonde (15), une unité de commande (20) d'entraînement configurée pour commander un état d'entraînement de l'unité d'entraînement (16) de sonde, et une unité de détection de quantité de mouvement (17) configurée pour détecter une quantité de mouvement de la sonde (15) déplacée par l'unité d'entraînement (16) de sonde ;
l'unité d'entraînement (16) de sonde inclut une fonction fournissant une vibration, configurée pour fournir à la sonde (15) une vibration ultrasonore et une fonction de mouvement, configurée pour déplacer la sonde (15) ; et
la sonde (15) étant en contact avec la peau du bout de doigt (10), l'unité d'entraînement (16) de sonde est amenée par un signal de commande provenant de l'unité de commande (20) d'entraînement à fournir au bout de doigt (10) un stimulus de mouvement accompagné d'un stimulus de vibration ultrasonore, et l'unité de détection de quantité de mouvement (17) est configurée pour détecter une quantité de mouvement de la sonde (15) à un instant où le sujet montre une réaction, de sorte qu'un seuil sensoriel cutané de bout du doigt (10) du sujet est mesuré,
dans lequel
l'unité d'entraînement (16) de sonde utilise un moteur ultrasonique (30) en tant qu'une source d'entraînement ; et
la sonde (15) est déplacée par une force d'entraînement du moteur ultrasonique (30), de sorte qu'une vibration ultrasonore issue du moteur ultrasonique (30) est fournie à la sonde (15), et un stimulus de mouvement accompagné d'un stimulus de vibration ultrasonore est fourni au bout de doigt (10).

2. Dispositif de mesure de seuil sensoriel cutané selon la revendication 1, dans lequel :
le moteur ultrasonique (30) configurant l'unité d'entraînement (16) de sonde inclut un élément piézoélectrique (32) qui est fléchi et déformé par application d'une tension, un arbre de vibration (33) qui vibre en réponse à la flexion et la déformation de l'élément piézoélectrique (32), et un corps mobile (34) qui est configuré pour pouvoir être déplacé par rapport à l'arbre de vibration (33) et se déplace linéairement le long de l'arbre de vibration (33) en réponse à la flexion et la déformation de l'élément piézoélectrique (32) ; et
la sonde (15) est fixée au corps mobile (34).

3. Dispositif de mesure de seuil sensoriel cutané selon la revendication 2, dans lequel :
l'unité d'entraînement (16) de sonde inclut deux des arbres de vibration (33) disposés en parallèle, l'élément piézoélectrique (32) connecté à une partie terminale de chacun des arbres de vibration (33), le corps mobile (34) fourni sur chacun des arbres de vibration (33), et un plateau (31) qui forme un pont entre l'un et l'autre des corps mobiles (34) ; et
la sonde (15) est fixée au plateau (31).

4. Dispositif de mesure de seuil sensoriel cutané selon l'une quelconque des revendications 1 à 3, dans lequel :
une fenêtre de contact (60) destinée à exposer la sonde (15) est ouverte en une position centrale du boîtier de corps (2), et la sonde (15) est configurée pour pouvoir être déplacée le long de la fenêtre de contact (60) ; et
la fenêtre de contact (60) est dotée d'une garniture (61) faite d'un corps élastique qui obture un espace entre un bord de l'ouverture de la fenêtre de contact (60) et un bord périphérique de la sonde (15).

5. Dispositif de mesure de seuil sensoriel cutané selon la revendication 4, comprenant une structure de montage destiné au montage et à la fixation du boîtier de corps (2) sur le bout de doigt (10) du sujet, la sonde (15) étant en contact avec une peau à travers la fenêtre de contact (60).

6. Dispositif de mesure de seuil sensoriel cutané selon l'une quelconque des revendications 1 à 5, dans lequel :
le boîtier de corps (2) loge en son sein une unité de communication (22) destinée à établir un état de connexion de communication sans fil avec un dispositif de commande principal (70) actionné par le sujet et dans lequel est installé un programme de commande ;
sur la base d'un signal de commande transmis depuis le dispositif de commande principal (70) via l'unité de communication (22), la sonde (15) est déplacée ; et
un seuil sensoriel cutané détecté par l'unité de détection de quantité de mouvement (17) est transmis vers le dispositif de commande principal (70) via l'unité de communication (22).
